(19) Europäisches Patentamt / European Patent Office / Office européen des brevets

(11) **EP 4 400 031 A1**

(12) **EUROPEAN PATENT APPLICATION**
published in accordance with Art. 153(4) EPC

(43) Date of publication:
**17.07.2024 Bulletin 2024/29**

(21) Application number: **22883165.7**

(22) Date of filing: **23.06.2022**

(51) International Patent Classification (IPC):
**A61B 3/135** $^{(2006.01)}$

(52) Cooperative Patent Classification (CPC):
**A61B 3/135**

(86) International application number:
**PCT/JP2022/025094**

(87) International publication number:
**WO 2023/067853 (27.04.2023 Gazette 2023/17)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.10.2021 JP 2021170020**

(71) Applicant: **TOPCON CORPORATION
Tokyo 174-8580 (JP)**

(72) Inventor: **MORISHIMA Syunichi
Tokyo 174-8580 (JP)**

(74) Representative: **Vossius & Partner
Patentanwälte Rechtsanwälte mbB
Siebertstrasse 3
81675 München (DE)**

(54) **OPHTHALMOLOGIC DEVICE**

(57)    This ophthalmologic device is provided with a light source, an iris diaphragm, a light condensing member, a slit, and an image sensor. The light source can be arranged at a position that is substantially optically conjugated with the iris of an eye to be examined. The iris diaphragm is arranged at a position that is substantially optically conjugated with the light source, in which an opening through which illumination light emitted from the light source can pass is formed at a position off-centered from the optical axis. The light condensing member is arranged between the light source and the iris diaphragm. The slit can be arranged at a position that is substantially optically conjugated with the fundus of an eye to be examined, in which a slit-like opening through which illumination light has passed through the opening can pass is formed. The image sensor receives return-light from the fundus irradiated with the illumination light that has passed through the slit.

FIG. 1

EP 4 400 031 A1

**Description**

[TECHNICAL FIELD]

The present invention relates to an ophthalmic apparatus.

[BACKGROUND ART]

In recent years, screening tests have been performed using ophthalmic apparatuses. Such ophthalmic apparatuses are expected to be applied to self-examinations, and further downsizing and weight saving of the ophthalmic apparatuses are desired.

For example, Patent Document 1 and Patent Document 2 disclose ophthalmic apparatuses configured to pattern-illuminate an eye to be examined using slit light and to detect returning light of the slit light using a CMOS (Complementary Metal Oxide Semiconductor) image sensor. These ophthalmic apparatuses can acquire images of the eye to be examined with a simple configuration, by adjusting the illumination pattern and the timing of light receiving timing using the CMOS image sensor.

[PRIOR ART DOCUMENTS]

[PATENT DOCUMENTS]

[0004]

　[Patent Document 1] US Patent No. 7831106
　[Patent Document 2] US Patent No. 8237835

[SUMMARY OF THE INVENTION]

[PROBLEMS TO BE SOLVED BY THE INVENTION]

In case of illuminating the eye to be examined using the slit light to acquire images of the eye to be examined, images with high efficiency and high contrast can be acquired by illuminating the eye to be examined with slit light having little unevenness.

The present invention has been made in view of such circumstances, and one of objects of the present invention is to provide a new technique for illuminating the eye to be examined with slit light having little unevenness.

[MEANS OF SOLVING THE PROBLEMS]

The first aspect of embodiments is an ophthalmic apparatus, including: a light source configured to be capable of being positioned at a position substantially conjugate optically to an iris of an eye to be examined; an iris aperture in which an aperture, through which illumination light from the light source passes, is formed at an eccentric position from an optical axis, and that is positioned at a position substantially conjugate optically to the light source; a condensing member positioned between the light source and the iris aperture; a slit in which a slit-shaped aperture, through which the illumination light having passed through the aperture passes, is formed, and that is configured to be capable of being positioned at a position substantially conjugate optically to a fundus of the eye to be examined; and an image sensor configured to receive returning light from the fundus illuminated by the illumination light having passed through the slit.

In the second aspect of the embodiments, in the first aspect, the aperture of the the iris aperture is positioned at a focal position of the condensing member.

In the third aspect of the embodiments, in the first aspect or the second aspect, two or more apertures are formed in the iris aperture.

In the fourth aspect of the embodiments, in the third aspect, the ophthalmic apparatus includes two or more condensing member arranged corresponding to each of the two or more apertures.

In the fifth aspect of the embodiments, in any one of the first aspect to the fourth aspect, the ophthalmic apparatus further includes a lens positioned at an eccentric position from an optical axis of the iris aperture.

In the sixth aspect of the embodiments, in the fifth aspect, the lens is positioned so that an optical axis of the lens passes through the aperture of the iris aperture.

In the seventh aspect of the embodiments, in the first aspect or the second aspect, two or more apertures are formed in the iris aperture, the ophthalmic apparatus further including two or more lenses as the condensing member arranged corresponding to each of the two or more apertures, and D-cut processing is applied to a peripheral part of each of two lenses adjacent to each other among the two or more lenses.

In the eighth aspect of the embodiments, in any one of the fifth aspect to the seventh aspect, the ophthalmic apparatus further includes a condenser lens configured to make the illumination light from the light source into approximately collimated light; and a relay lens positioned between the iris aperture and the slit. For a first direction corresponding to a longitudinal direction of the slit-shaped aperture, when a length of the slit is Lx, a focal distance of the relay lens is f1, a focal distance of the lens is f2, a focal distance of the condenser lens is f3, and an emitted half-value at half angle of the light source is $\Theta$, a following formula (1) is satisfied.

[Equation 1]

$$f3 \times \tan\theta > \left(\frac{Lx}{2} \times \frac{f2}{f1}\right) \qquad (1)$$

For a second direction corresponding to a shorter direction of the slit-shaped aperture, when a length of the slit is Ly and an amount of eccentricity of the aperture relative to an optical axis is h, following formulas (2) to (4) are satisfied.

[Equation 2]

$$f3 \times \tan\theta > \left(\frac{Ly}{2} \times \frac{f2}{f1} + h\right) \qquad (2)$$

$$\frac{Dy}{2} < h \qquad (3)$$

$$Dy = Ly \times \frac{f2}{f1} \qquad (4)$$

In the ninth aspect of the embodiments, in any one of the first aspect to the fourth aspect, the condensing member includes a prism having deflected surfaces for the number of apertures formed in the iris aperture, and each of the deflected surfaces deflecting the illumination light toward corresponding aperture; and a lens positioned between the prism and the iris aperture.

In the tenth aspect of the embodiments, in any one of the first aspect to the ninth aspect, the ophthalmic apparatus further includes an optical scanner configured to deflect the illumination light having passed through the slit and to guide the deflected illumination light to the fundus. The ophthalmic apparatus is configured to capture light receiving result of the returning light acquired by the image sensor in synchronization with a deflection control for the optical scanner.

In the eleventh aspect of the embodiments, in any one of the first aspect to the tenth aspect, the ophthalmic apparatus further includes a hole mirror in which a hole is formed at a position substantially conjugate optically to the iris of the eye to be examined, and configured to couple an optical path of the illumination light having passed through the slit with an optical path of returning light from the fundus.

In the twelfth aspect of the embodiments, in any one of the first aspect to the eleventh aspect, the ophthalmic apparatus further includes a movement mechanism configured to move the slit in an optical axis direction according to a state of the eye to be examined.

It should be noted that the configurations according to a plurality of aspects described above can be combined arbitrarily.

[EFFECTS OF THE INVENTION]

According to the present invention, a new technique for illuminating an eye to be examined with slit light having little unevenness can be provided.

[BRIEF EXPLANATION OF THE DRAWINGS]

[FIG. 1] FIG. 1 is a schematic diagram illustrating an example of a configuration of an optical system of an ophthalmic apparatus according to embodiments.
[FIG. 2] FIG. 2 is a schematic diagram illustrating an example of a configuration of an optical system of the ophthalmic apparatus according to the embodiments.

[FIG. 3] FIG. 3 is a schematic diagram illustrating a broad outline of a configuration of an optical system of the ophthalmic apparatus according to the embodiments.

[FIG. 4] FIG. 4 is a schematic diagram illustrating a broad outline of a configuration of an optical system of the ophthalmic apparatus according to the embodiments.

[FIG. 5] FIG. 5 is a schematic diagram illustrating a broad outline of a configuration of an optical system of the ophthalmic apparatus according to the embodiments.

[FIG. 6] FIG. 6 is a schematic diagram illustrating a broad outline of a configuration of an optical system of the ophthalmic apparatus according to the embodiments.

[FIG. 7] FIG. 7 is a diagram explaining an operation of the ophthalmic apparatus according to the embodiments.

[FIG. 8] FIG. 8 is a diagram explaining an operation of the ophthalmic apparatus according to the embodiments.

[FIG. 9] FIG. 9 is a diagram explaining an operation of the ophthalmic apparatus according to the embodiments.

[FIG. 10] FIG. 10 is a schematic diagram illustrating an example of a configuration of a control system of the ophthalmic apparatus according to the embodiments.

[FIG. 11] FIG. 11 is a flowchart illustrating an example of an operation of the ophthalmic apparatus according to the embodiments.

[FIG. 12] FIG. 12 is a schematic diagram illustrating an example of a configuration of an optical system of an ophthalmic apparatus according to a first modification example of the embodiments.

[FIG. 13] FIG. 13 is a schematic diagram illustrating an example of a configuration of an optical system of an ophthalmic apparatus according to a second modification example of the embodiments.

[FIG. 14] FIG. 14 is a schematic diagram illustrating a broad outline of a configuration of an optical system of the ophthalmic apparatus according to the second modification example of the embodiments.

[MODES FOR CARRYING OUT THE INVENTION]

Referring now to the drawings, exemplary embodiments of an ophthalmic apparatus according to the present invention are described below. The contents of the document cited in the present specification can be appropriately incorporated as contents of the following embodiments.

An ophthalmic apparatus according to embodiments is configured to be capable of acquiring images of an eye to be examined using slit-scan method using a slit-shaped illumination light. Specifically, the ophthalmic apparatus is configured to illuminate a predetermined site of the eye to be examined while moving a slit-shaped irradiated position (irradiated range) using an optical scanner, and to receive returning light from the predetermined site using an image sensor with a one-dimensional or two-dimensional array of light receiving elements. Light receiving result of the returning light is read out from the light receiving element(s) at light receiving position of the returning light corresponding to the irradiated position of the illumination light, in synchronization with the movement timing of the irradiated position of the illumination light.

The ophthalmic apparatus according to the embodiments includes an iris aperture provided on an illumination side and a condensing member positioned so as to condense light on an aperture formed in the iris aperture. This allows the illumination light emitted from the light source to be efficiently irradiated to the slit for generating slit-shaped illumination light. Furthermore, the slit can be illuminated evenly by the illumination light having passed through the aperture of the iris aperture. As a result, the eye to be examined can be illuminated with the illumination light with high efficiency, and images of the eye to be examined with high contrast can be acquired.

In some embodiments, the predetermined site is an anterior segment or a posterior segment. Examples of the anterior segment include a cornea, an iris, a crystalline lens, a ciliary body, and a ciliary zonule. Examples of the posterior segment include a vitreous body, and a fundus or the vicinity of the fundus (retina, choroid, sclera, etc.).

A method of controlling the ophthalmic apparatus according to the embodiments includes one or more steps for realizing the processing executed by a processor (computer) in the ophthalmic apparatus according to the embodiments. A program according to the embodiments causes the processor to execute each step of the method of controlling the ophthalmic apparatus according to the embodiments. A recording medium according to the embodiments is a non-transitory recording medium (storage medium) on which the program according to the embodiments is recorded.

The term "processor" as used herein refers to a circuit such as, for example, a central processing unit (CPU), a graphics processing unit (GPU), an application specific integrated circuit (ASIC), and a programmable logic device (PLD). Examples of PLD include a simple programmable logic device (SPLD), a complex programmable logic device (CPLD), and a field programmable gate array (FPGA). The processor realizes, for example, the function according to the embodiments by reading out a computer program stored in a storage circuit or a storage device and executing the computer program.

Hereinafter, a case where the ophthalmic apparatus according to the embodiments acquires images of the fundus of the eye to be examined mainly will be described.

Hereinafter, it is assumed that an X direction is a direction orthogonal to an optical axis direction (left and right direction, horizontal direction) of an objective lens, and a Y direction is a direction orthogonal to the optical axis direction (upper and lower direction, vertical direction) of the objective lens. AZ direction is assumed to be the optical axis direction of the objective lens.

[Configuration of optical system]

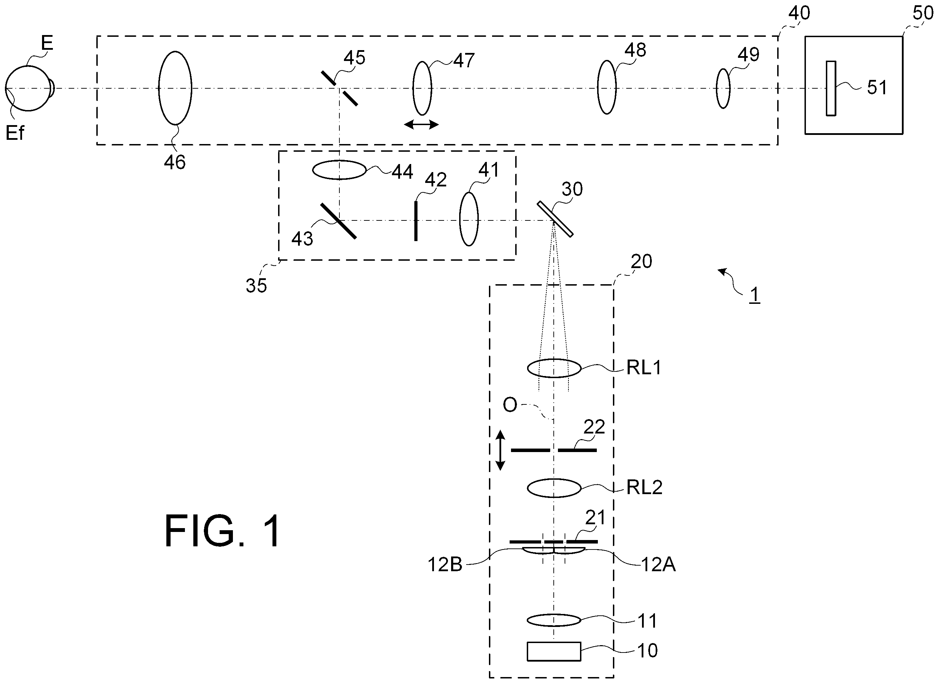

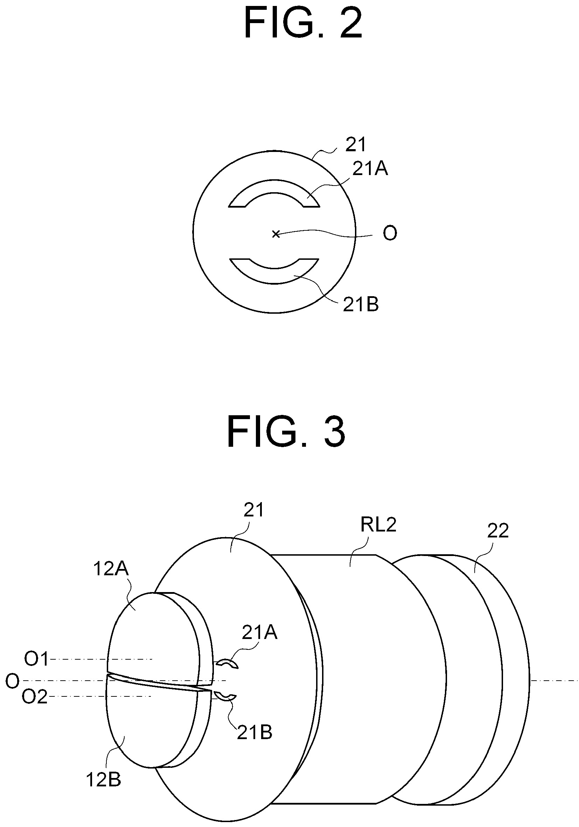

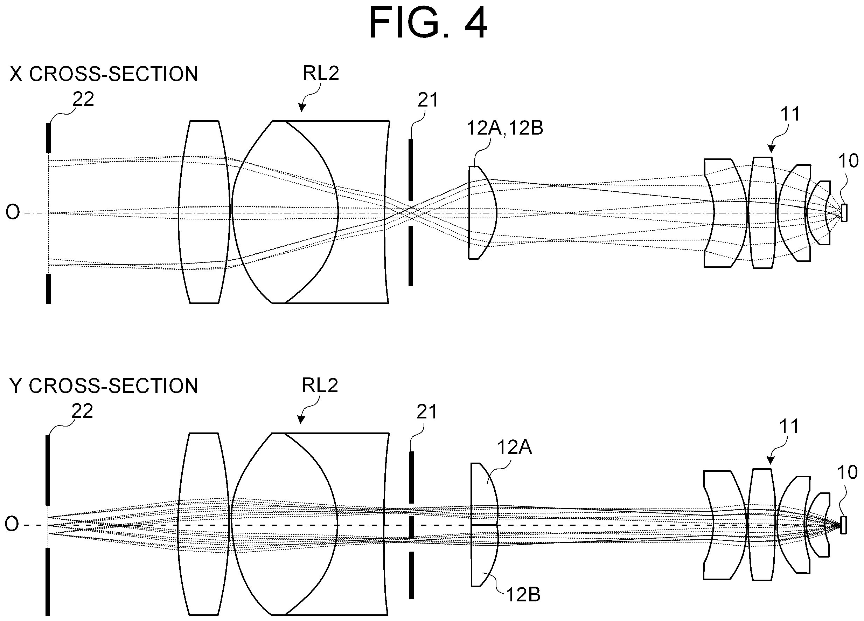

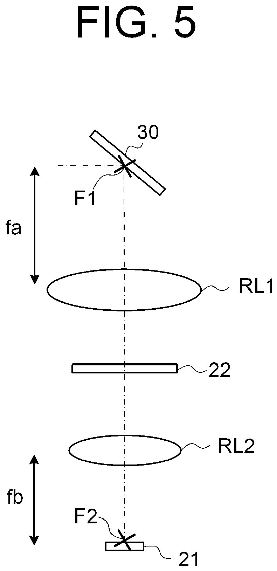

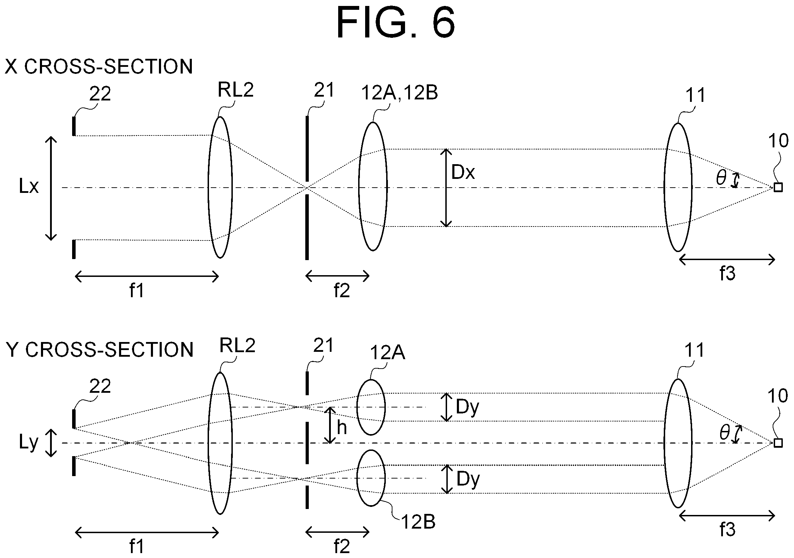

FIGs. 1 to 6 illustrate an example of a configuration of an optical system of the ophthalmic apparatus according to the embodiments. FIG. 1 represents an example of the configuration of the optical system of the ophthalmic apparatus 1 according to the embodiments. FIG. 2 schematically represents an example of a configuration of an iris aperture 21 in FIG. 1 when viewed from a direction of an optical axis O. FIG. 3 schematically represents an example of an arrangement of eccentric lenses 12A and 12B in FIG. 1, three-dimensionally. FIG. 4 schematically represents an example of a configuration of the eccentric lenses 12A and 12B in the X and Y cross-sections. FIG. 5 represents a broad outline of configurations of relay lens systems RL1 and RL2 in FIG. 1. FIG. 6 represents a diagram explaining an arrangement of the optical system from a light source 10 to a slit 22 in FIG. 1. In FIGs. 1 to 6, like parts are designated by like reference numerals as in repetitious description of such parts may not be provided.

The ophthalmic apparatus 1 includes an illumination optical system 20, an optical scanner 30, a projection optical system 35, an imaging optical system 40, and an imaging device 50. In some embodiments, the light source 10 is provided outside of the illumination optical system 20. In some embodiments, the illumination optical system 20 includes at least one of the optical scanner 30 or the projection optical system 35. In some embodiments, the imaging optical system 40 includes the imaging device 50. In some embodiments, the projection optical system 35 or the illumination optical system 20 includes the optical scanner 30.

(Illumination optical system 20)

The illumination optical system 20 generates slit-shaped illumination light using light from the light source 10, and guides the generated illumination light to the optical scanner 30.

The illumination optical system 20 includes the light source 10, a condenser lens 11, the eccentric lenses 12A and 12B, the iris aperture 21, the relay lens system RL2, the slit 22, and the relay lens system RL1.

(Light source 10)

The light source 10 is positioned at a position substantially conjugate optically to an iris (pupil) of the eye to be examined. The light source 10 includes a visible light source that generates light in the visible region. For example, the light source 10 generates light having a central wavelength in the wavelength range of 420 nm to 700 nm. This type of light source 10 includes, for example, an LED (Light Emitting Diode), an LD (Laser Diode), a halogen lamp, or a xenon lamp. In some embodiments, the light source 10 includes a white light source or a light source capable of outputting light with each color component of RGB. In some embodiments, the light source 10 includes a light source capable of switching to output the light in infrared region or the light in visible region. In some embodiments, the light source 10 includes a phosphor (central fluorescence wavelength 550 nm), a first LD (central wavelength 450 nm) that emits excitation light to excite the phosphor, a second LED (central wavelength 450 nm), and an optical path coupling member. The optical path coupling member outputs white light by coupling the emitted light from the phosphor excited by the first LED and the emitted light from the second LED.

(Condenser lens 11)

The condenser lens 11 condenses the light emitted from light source 10. The light source 10 is positioned at a focal position of the condenser lens 11, and the light emitted from the light source 10 is made into collimated light and is guided to the eccentric lenses 12A and 12B.

(Iris aperture 21)

The iris aperture 21 (specifically, aperture(s) described below) is configured to be capable of being positioned at a position substantially conjugate optically to the iris (pupil) of an eye E to be examined. In the iris aperture 21, one or more apertures are formed at position(s) away from the optical axis O.

FIG. 2 shows a broad outline of a configuration of the iris aperture 21.

The apertures 21A and 21B having a predetermined thickness along a circumferential direction centered on the optical axis O are formed in the iris aperture 21. The aperture formed in the iris aperture 21 defines an incident position (incident shape) of the illumination light on the iris of the eye E to be examined. For example, by forming the apertures 21A and 21B as shown in FIG. 2, when the pupil center of the eye E to be examined is arranged on the optical axis O, the illumination light can enter into the eye from positions deviated from the pupil center (specifically, point-symmetrical positions centered on the pupil center).

In FIG. 2, the apertures 21A and 21B are formed in the iris aperture 21. However, the embodiments are not limited to the number of apertures formed in the iris aperture 21. For example, a single aperture may be formed in the iris aperture 21 or three or more apertures may be formed in the iris aperture 21.

(Eccentric lenses 12A and 12B)

As shown in FIG. 1, each of the eccentric lenses 12A and 12B is a lens positioned at an eccentric position from the optical axis O (the optical axis of the iris aperture 21) and functions as a condensing member. The eccentric lenses 12A and 12B are arranged between the condenser lens 11 (light source 10) and the iris aperture 21. The eccentric lenses 12A and 12B are arranged corresponding to the apertures 21A and 21B formed in the iris aperture 21, respectively. When "n" ("n" is an integer greater than or equal to 2) apertures are formed in the iris aperture 21, "n" (pieces of) eccentric lenses, each of which corresponds to each of the "n" (piece of) apertures, are arranged between the condenser lens 11 and the iris aperture 21.

In FIG. 1, the eccentric lens 12A is positioned at a position eccentric from the optical axis O of the illumination optical system 20 so that the eccentric lens 12A faces to the aperture 21A of the iris aperture 21. Further, the aperture 21A of the iris aperture 21 is arranged at a focal position of the eccentric lens 12A. In other words, the eccentric lens 12A condenses the illumination light from condenser lens 11 on the aperture 21A.

Similarly, the eccentric lens 12B is positioned at a position eccentric from the optical axis O of the illumination optical system 20 so that the eccentric lens 12B faces to the aperture 21B of the iris aperture 21. Further, the aperture 21B of the iris aperture 21 is arranged at a focal position of the eccentric lens 12B. In other words, the eccentric lens 12B condenses the illumination light from condenser lens 11 on the aperture 21B.

FIG. 3 schematically shows a three-dimensional positional relationship between the iris aperture 21 and the eccentric lenses 12A and 12B. In FIG. 3, like reference numerals designate like parts as in FIG. 1 and FIG. 2. The same description may not be repeated. FIG. 4 schematically represents an example of a configuration of the eccentric lenses 12A and 12B in the X and Y cross-sections. In FIG. 4, parts similar to those in FIGs. 1 to 3 are denoted by the same reference symbols, and description thereof is omitted as appropriate.

The eccentric lens 12A is arranged so that an optical axis O1 of the eccentric lens 12A passes through the aperture 21A of the iris aperture 21. Further, the eccentric lens 12B is arranged so that an optical axis O2 of the eccentric lens 12B passes through the aperture 21B of the iris aperture 21.

For example, it is assumed that the apertures 21A and 21B are formed at point-symmetrical positions with reference to the optical axis O along a circumference centered on the optical axis O. In this case, the optical axis O1 is arranged so that, on the iris aperture 21, the Y direction is shifted in the +Y direction by a radius of the circumference from the position of the optical axis O and the X direction coincides with a position of the center of gravity in a longitudinal direction of the aperture 21A. Similarly, the optical axis O2 is arranged so that, on the iris aperture 21, the Y direction is shifted in the -Y direction by the radius of the circumference from the position of the optical axis O and the X direction coincides with a position of the center of gravity in a longitudinal direction of the aperture 21B.

In some embodiments, D-cut processing is applied to each of peripheral parts of two eccentric lenses adjacent to each other among the eccentric lenses arranged corresponding to the apertures formed in the iris aperture 21.

As shown in FIG. 3 and FIG. 4, the eccentric lens 12A is an eccentric D-cut lens. The D-cut processing is applied to the peripheral part, which is adjacent to the eccentric lens 12B, of the eccentric lens 12A. Further, the eccentric lens 12B is an eccentric D-cut lens. The D-cut processing is applied to the peripheral part, which is adjacent to the eccentric lens 12A, of the eccentric lens 12B. The D-cut processing may be applied to only the peripheral part of one of the eccentric lens 12A or the eccentric lens 12B.

In some embodiments, a worked surface of the eccentric lens 12A is arranged so as to be in contact with a work surface of the eccentric lens 12B. In some embodiments, the worked surface of the eccentric lens 12A is arranged at a predetermined distance from the worked surface of the eccentric lens 12B.

(Slit 22)

As shown in FIG. 1, the slit (fundus slit) 22 (specifically, aperture(s) described below) is configured to be capable of being positioned at a position substantially conjugate optically to a fundus Ef of the eye E to be examined. For example, in the slit 22, an aperture (slit-shaped aperture) is formed extending in a direction corresponding to a line direction (row direction) that is read out from the image sensor 51 described below using the rolling shutter method. The aperture formed in the slit 22 defines an irradiated pattern of the illumination light on the fundus Ef of the eye E to be examined.

The slit 22 can be moved in the optical axis direction of the illumination optical system 20 using a movement mechanism (movement mechanism 22D described below). The movement mechanism moves the slit 22 in the optical axis direction, under the control from a controller 100 described below. For example, the controller 100 controls the movement mechanism in accordance with a state of the eye E to be examined. This allows to move the position of the slit 22 in accordance with the state (specifically, the dioptric power or the shape of the fundus Ef) of the eye E to be examined.

In some embodiments, the slit 22 is configured so that at least one of the position of the aperture or the shape of the aperture can be changed in accordance with the state of the eye E to be examined without being moved in the optical axis direction. The function of the slit 22 with these functions is, for example, realized by a liquid crystal shutter.

(Relay lens system RL1, RL2)

In FIG. 1, the relay lens system RL1 is arranged between the optical scanner 30 and the slit 22, and the relay lens system RL2 is arranged between the slit 22 and the iris aperture 21.

FIG. 5 schematically shows a broad outline of a configuration of the relay lens systems RL1 and RL2 according to the embodiments. In FIG. 5, parts similar to those in FIG. 1 are denoted by the same reference symbols, and description thereof is omitted as appropriate.

The relay lens system RL1 includes one or more lenses. A back focal position F1 of the relay lens system RL1 is positioned at a position substantially conjugate optically to the iris of the eye E to be examined.

As described below, the optical scanner 30, that is arranged at a position substantially conjugate optically to the iris of the eye E to be examined, is arranged at the back focal position F1 of the relay lens system RL1 or the vicinity of the back focal position F1. Therefore, even when the slit 22 is moved in the optical axis direction in accordance with the state (dioptric power) of the eye E to be examined, a size of a slit image (image formed by the light having passed through the aperture formed in the slit 22) projected onto the fundus Ef does not change, regardless of the state of the eye E to be examined. This means that the projection magnification of the slit image onto the fundus Ef does not change even when the slit 22 moves in the optical axis direction.

That is, by arranging the optical scanner 30 at the back focal position F1 (or the vicinity of the back focal position F1) of the relay lens system RL1, the Badal optical system is configured with the relay lens system RL1, the relay lenses 41 and 44, and the objective lens 46 (see FIG. 1).

This allows to keep the projected angle of view (projection magnification) of the slit image with reference to the visual axis of the eye to be examined (longitudinal and shorter directions of the slit 22) constant, regardless of the state (dioptric power) of the eye E to be examined. As a result, the size of the slit image does not change regardless of the state of the eye E to be examined. This allows to keep the deflection operation speed of the optical scanner 30 constant, and to simplify the control of the optical scanner 30.

In addition, since the projected angle of view (projection magnification) of the slit image with reference to the visual axis of the eye E to be examined is constant regardless of the state (dioptric power, or the like) of the eye E to be examined, the illumination intensity of the slit image at the fundus Ef can be kept constant.

Further, in case of acquiring images at a predetermined imaging angle of view in the ophthalmic apparatus, since the projection magnification is constant as described above, this eliminates the need for a margin of the length in the longitudinal length of the slit 22 provided to acquire a slit image of a predetermined size.

Further, in the same way as the relay lens system RL1, the relay lens system RL2 includes one or more lenses. The iris aperture 21 (apertures) is arranged at a front focal position F2 of the relay lens system RL2 or the vicinity of the front focal position F2.

As described above, the back focal position F1 of the relay lens system RL1 and the front focal position F2 of the relay lens system RL2 are positions substantially conjugate optically to the iris of the eye to be examined. Therefore, the projection magnification from the iris aperture 21 to the optical scanner 30 (arranged at the back focal position F1) is determined by a focal distance fa of the relay lens system RL1 and a focal distance fb of the relay lens system RL2. In this case, the projection magnification is (fa / fb).

The ophthalmic apparatus according to the embodiments is required to form images of the iris aperture 21 with a predetermined size on the iris of the eye E to be examined. When the projection magnification from the iris of the eye E to be examined to the optical scanner 30 via the objective lens 46 is a known projection magnification, an image of the iris aperture 21 of a predetermined size should be projected on the optical scanner 30. In this case, the projection magnification from the iris aperture 21 to the optical scanner 30 is determined by the focal distance fa of the relay lens system RL1 and the focal distance fb of the relay lens system RL2. Therefore, by changing at least one of the focal distance fa or the focal distance fb, the image of the iris aperture 21 can be easily formed on the iris of the eye E to be examined with a predetermined size. In some embodiments, while the focal distance fa remains fixed, the focal distance fb is changed alone.

The focal distance fa is a composite focal distance of the relay lens system RL1. In some embodiments, the relay lens system RL1 includes a plurality of the lenses with different dioptric powers, and changes the focal distance fa by changing at least one of the lenses constituting the relay lens system RL1. In some embodiments, at least one of the lenses constituting the relay lens system RL1 is a lens whose dioptric power can be changed. Examples of the lens whose dioptric power can be changed include a liquid crystal lens, a liquid lens, and an Alvarez lens. Even when the focal distance fa is changed, the back focal position of the relay lens system RL1 is arranged at a position substantially conjugate optically to the iris (pupil conjugate position) of the eye E to be examined.

The focal distance fb is a composite focal distance of the relay lens system RL2. In some embodiments, the relay lens system RL2 includes a plurality of the lenses with different dioptric powers, and changes the focal distance fb by changing at least one of the lenses constituting the relay lens system RL2. In some embodiments, at least one of the lenses constituting the relay lens system RL2 is a lens whose dioptric power can be changed. Even when the focal distance fb is changed, the front focal position of the relay lens system RL2 is also arranged at a position substantially conjugate optically to the iris (pupil conjugate position) of the eye E to be examined.

In addition, for imaging the fundus Ef, it is desirable to use a light source that emits a high-intensity light. However, light sources available for general use (light sources that are mass-produced) are limited in the size of the emitting surface (luminous area, output luminous flux cross section size). Thereby, the image of the iris aperture 21 should be projected onto the optical scanner 30 with a projection magnification corresponding to the size of the emitting surface of the light source.

According to the present embodiments, by changing at least one of the focal distance fa or the focal distance fb, the projecting magnification from the iris aperture 21 to the optical scanner 30 can be changed. Thereby, the image of the iris aperture 21 with any size can be projected onto the optical scanner 30 with the desired size. This allows to project the image of the iris aperture 21 with a desired size onto the optical scanner 30 by simply changing at least one of the focal distance fa or the focal distance fb even when the size of the emitting surface of the light source is different, and to improve the degree of freedom in designing optical systems. In particular, this allows to fix the movement amount of the slit 22 in response to changes in the dioptric power of the eye E to be examined (sensitivity of the movement of the slit 22 in response to changes in the dioptric power) by fixing the focal distance fa and changing the focal distance fb alone, and to further improve the degree of freedom in designing optical systems.

Further, the effective diameter of one or more lenses constituting the relay lens system RL1 can be reduced in the configuration shown in FIG. 1.

The reason for this is that the slit 22, that is arranged at a position substantially conjugate optically to the fundus Ef of the eye E to be examined, is arranged between the optical scanner 30 and the iris aperture 21. The slit 22 can be moved in the optical axis direction in accordance with the dioptric power of the eye E to be examined. Here, the projection magnification from the iris aperture 21 to the optical scanner 30 is determined by the first distance, that is a distance between the optical scanner 30 and the relay lens system RL1, and the second distance, that is a distance between the iris aperture 60 and the relay lens system RL1. Thereby, when the first distance is shortened, the second distance should also be shortened. However, since it is necessary to maintain the conjugate relationship with the iris and the conjugate relationship with the fundus Ef while securing the space for movement of the slit 22 in the optical axis direction, the first distance becomes longer and the effective diameter of the relay lens system RL1 becomes larger. According to the present embodiments, by providing the relay lens system RL2, the projection magnification can be adjusted using the relay lens system RL2 even if the first distance is shortened. This allows to shorten the first distance while maintaining the conjugate relationship with the iris and the conjugate relationship with the fundus Ef and securing the space for movement of the slit 22 in the optical axis direction, and to reduce the effective diameter of the one or more lenses constituting the relay lens system RL1.

Further, since the effective diameter of the one or more lenses constituting the relay lens system RL1 can be reduced, the length of the optical system from the optical scanner 30 to the light source 10 can be reduced.

In the illumination optical system 20 with this configuration, the light emitted from the light source 10 is transmitted through the condenser lens 11, is transmitted through the eccentric lenses 12A and 12B, and passes through the apertures 21A and 21B formed in the iris aperture 21. The light that has passed through the apertures 21A and 21B formed in the iris aperture 21 is transmitted through the relay lens system RL2, and is output as the slit-shaped illumination light by passing through the apertures formed in the slit 22. The slit-shaped illumination light is transmitted through the relay lens system RL1, and is guided to the optical scanner 30.

In the embodiments, in order to perform efficient illumination with slit-shaped illumination light, it is desirable to satisfy the following conditions.

FIG. 6 schematically represents the optical system from the light source 10 to the slit 22 in FIG. 1. In FIG. 6, parts similar to those in FIGs. 1 to 5 are denoted by the same reference symbols, and description thereof is omitted as appropriate. In FIG. 6, for convenience of explanation, it is assumed that, for the aperture(s) formed in slit 22, the longitudinal direction is the X direction and the shorter direction is the Y direction.

Here, it is assumed that a back focal distance of the relay lens system RL2 is f1 (in FIG. 5, focal distance fb), respective back focal distances of the eccentric lenses 12A and 12B are f2, and a front focal distance of the condenser lens 11 is f3. Further, it is assumed that an emitted half-value at half angle from the light source 10 is θ, and an amount (height) of eccentricity of the apertures 21A and 21B of the iris aperture 21 relative to the optical axis O is h.

First, in the X direction (here, the longitudinal direction), the following applies.

As described above, the iris aperture 21 is positioned at a position substantially conjugate optically to the light source 10. Further, the slit 22 is positioned at a position substantially conjugate optically to a lens intermediate position midway between the eccentric lenses 12A and 12B and the condenser lens 11. In this case, when the image of the slit 22 is relayed to the lens intermediate position using the relay lens system RL2 and the eccentric lens 12A (or eccentric lens 12B), the projection magnification is "f2 / f1". Therefore, when a length of the aperture formed in slit 22 for the X direction is Lx, an illumination light beam diameter Dx at the lens intermediate position required to illuminate the length Lx of the aperture formed in slit 22 can be expressed by a following formula (5).

[Equation 3]

$$Dx = Lx \times \left(\frac{f2}{f1}\right) \qquad (5)$$

In order to illuminate the illumination light beam diameter Dx described above, it is desirable that a light beam diameter of the condenser lens 11 should satisfy a following formula (6).

[Equation 4]

$$f3 \times \tan\theta > \frac{Dx}{2} = \left(\frac{Lx}{2} \times \frac{f2}{f1}\right) \qquad (6)$$

In formula (6), the length Lx of the slit 22 and the front focal distance f1 of the relay lens system RL2 can be determined from the functions (specifications) to be realized by the ophthalmic apparatus 1. In addition, the emitted half-value at half angle θ can be determined from the functions (specifications) to be realized by the light source 10.

Similarly, in the Y direction (here, the shorter direction), the following applies.

When the image of the slit 22 is relayed to the lens intermediate position using the relay lens system RL2 and the eccentric lens 12A (or eccentric lens 12B), the projection magnification is "f2 / f1". Therefore, when a length of the aperture formed in slit 22 for the Y direction is Ly, an illumination light beam diameter Dy at the lens intermediate position required to illuminate the length Ly of the aperture formed in slit 22 can be expressed by a following formula (7).

[Equation 5]

$$Dy = Ly \times \left(\frac{f2}{f1}\right) \qquad (7)$$

In order to illuminate the illumination light beam diameter Dy described above, it is desirable that a light beam diameter of the condenser lens 11 should satisfy a following formula (8).

[Equation 6]

$$f3 \times \tan\theta > \left(\frac{Dy}{2} + h\right) = \left(\frac{Ly}{2} \times \frac{f2}{f1} + h\right) \qquad (8)$$

In addition, in order to avoid overlapping of the two illumination light beam diameters arranged in the Y direction, it is desirable that a following formula (9) should be satisfied.

[Equation 7]

$$\frac{Dy}{2} < h \qquad (9)$$

In formula (8) and formula (9), the length Ly of the slit 22, the front focal distance f1 of the relay lens system RL2, and the amount of eccentricity h can be determined from the functions (specifications) to be realized by the ophthalmic apparatus 1. The emitted half-value at half angle θ is determined from the functions (specifications) to be realized by the light source 10.

As described above, it is desirable to determine the back focal distance f2 of the eccentric lenses 12A and 12B and the front focal distance f3 of the condenser lens 11 so that formula (6) is satisfied in the X direction and formulas (8) to (9) are satisfied in the Y direction. This allows the slit 22 to be efficiently illuminated evenly with the illumination light without increasing the size of the optical system.

(Optical scanner 30)

The optical scanner 30 is positioned at a position substantially conjugate optically to the iris of the eye E to be examined. The optical scanner 30 deflects the slit-shaped illumination light transmitted through the relay lens system RL1 (slit-shaped light having passed through the aperture formed in the slit 22). Specifically, the optical scanner 30 deflects the slit-shaped illumination light for sequentially illuminating a predetermined illuminated range of the fundus Ef to guide the illumination light to the projection optical system 35, while changing a deflection angle within a predetermined deflection angle range with the iris or the vicinity of the iris of the eye E to be examined as a scan center position. The optical scanner 30 can deflect the illumination light one-dimensionally or two-dimensionally.

In case that the optical scanner 30 deflects the illumination light one-dimensionally, the optical scanner 30 includes a galvano scanner that deflects the illumination light within a predetermined deflection angle range with reference to a predetermined deflection direction. In case that the optical scanner 30 deflects the illumination light two-dimensionally, the optical scanner 30 includes a first galvano scanner and a second galvano scanner. The first galvano scanner deflects the illumination light so as to move the irradiated position of the illumination light in a horizontal direction orthogonal to the optical axis of the illumination optical system 20. The second galvano scanner deflects the illumination light deflected by the first galvano scanner so as to move the irradiated position of the illumination light in a vertical direction orthogonal to the optical axis of the illumination optical system 20. Examples of scan mode for moving the irradiated position of the illumination light using the optical scanner 30 include a horizontal scan, a vertical scan, a cross scan, a radial scan, a circle scan, a concentric scan, and a helical (spiral) scan.

(Projection optical system 35)

The projection optical system 35 guides the illumination light deflected by the optical scanner 30 to the fundus Ef of the eye E to be examined. In the embodiments, the projection optical system 35 guides the illumination light deflected by the optical scanner 30 to the fundus Ef through an optical path coupled with an optical path of the imaging optical system 40 by a hole mirror 45 as an optical path coupling member described below.

The projection optical system 35 includes a relay lens 41, a black point plate 42, a reflective mirror 43, and a relay lens 44. Each of the relay lenses 41 and 44 includes one or more lenses.

(Black point plate 42)

The black point plate 42 is arranged at a position substantially conjugate optically to a lens surface of an objective lens 46 or the vicinity of the lens surface of the objective lens 46. This prevents the reflected light from the lens surface of the objective lens 46 from being guided to the light source 10.

In the projection optical system 35 with this configuration, the illumination light deflected by the optical scanner 30 is transmitted through the relay lens 41, passes through the black point plate 42, is reflected by the reflective mirror 43 toward the relay lens 44, is transmitted through the relay lens 44, and is guided to the hole mirror 45.

(Imaging optical system 40)

The imaging optical system 40 guides the illumination light that has been guided through the projection optical system 35 to the fundus Ef of the eye E to be examined, and also guides the returning light of the illumination light from the fundus Ef to the imaging device 50.

In the imaging optical system 40, an optical path of the illumination light from the projection optical system 35 and an optical path of the returning light of the illumination light from the fundus Ef are coupled. By using the hole mirror 45 as the optical path coupling member to couple these optical paths, pupil division between the illumination light and the returning light of the illumination light can be performed.

The imaging optical system 40 includes the hole mirror 45, the objective lens 46, a focusing lens 47, a relay lens 48, and an imaging lens 49. The relay lens 48 includes one or more lenses.

(Hole mirror 45)

In the hole mirror 45, the hole is formed. The hole is arranged on the optical axis of the imaging optical system 40. The hole of the hole mirror 45 is configured to be capable of being positioned at a position substantially conjugate optically to the iris of the eye E to be examined. The hole mirror 45 reflects the illumination light from the projection optical system 35 toward the objective lens 46, on the peripheral region of the hole. The hole mirror 45 with this configuration functions as an imaging aperture (photographic stop (diaphragm)).

Further, the hole mirror 45 functions as the optical path coupling member that couples an optical path of the illumination light that has passed through the slit 22 with an optical path of the returning light of the illumination light from the fundus Ef. That is, the hole mirror 45 is configured to couple the optical path of the illumination optical system 20 (projection optical system 35) and the optical path of the imaging optical system 40 arranged in a direction of the optical axis passing through the hole, and also to guide the illumination light reflected on the peripheral region of the hole to the fundus Ef.

(Focusing lens 47)

The focusing lens 47 can be moved in an optical axis direction of the imaging optical system 40 using a movement mechanism (not shown). The movement mechanism moves the focusing lens 47 in the optical axis direction under the control from the controller 100 described below. This allows to image the returning light of the illumination light having passed through the hole of the hole mirror 45 on the light receiving surface of the image sensor 51 in the imaging device 50 in accordance with the state of the eye E to be examined.

In the imaging optical system 40 with this configuration, the illumination light from the projection optical system 35 is reflected on the peripheral region of the hole formed in the hole mirror 45 toward the objective lens 46. The illumination light reflected on the peripheral region of the hole mirror 45 is refracted by the objective lens 46, enters into the eye through the pupil of the eye E to be examined, and illuminates the fundus Ef of the eye E to be examined.

The returning light of the illumination light from the fundus Ef is refracted by the objective lens 46, passes through the hole of the hole mirror 45, is transmitted through the focusing lens 47, is transmitted through the relay lens 48, and is imaged on the light receiving surface of the image sensor 51 in the imaging device 50 through the imaging lens 49.

(Imaging device 50)

The imaging device 50 includes the image sensor 51 that receives the returning light of the illumination light that has been guided from the fundus Ef of the eye E to be examined through the imaging optical system 40. The imaging device 50 can perform readout control of the light receiving result of the returning light under the control from the controller 100 described below.

(Image sensor 51)

The image sensor 51 realizes the function as a pixelated photodetector. The light receiving surface (detecting surface, imaging surface) of the image sensor 51 can be arranged at a position substantially conjugate optically to the fundus Ef.

The light receiving result(s) obtained using the image sensor 51 is/are read out using a rolling shutter method under the control from the controller 100 described below.

The image sensor 51 with this configuration includes a CMOS image sensor. In this case, the image sensor 51 includes a plurality of pixels (light receiving elements). The plurality of pixels includes a plurality of pixel groups arranged in a column direction. Each of the plurality of pixel groups includes pixels arranged in a row direction. Specifically, the image sensor 51 includes a plurality of pixels arranged two-dimensionally, a plurality of vertical signal lines, and a horizontal signal line. Each pixel includes a photodiode (light receiving element), and a capacitor. The vertical signal lines are provided for each pixel group in the column direction (vertical direction) orthogonal to the row direction (horizontal direction). Each of the vertical signal lines is selectively electrically connected to the pixel group in which the electrical charge corresponding to the light receiving result is accumulated. The horizontal signal line is selectively electrically connected to the vertical signal lines. Each of the pixels accumulates the electrical charge corresponding to the light receiving result of the returning light. The accumulated electrical charge is read out sequentially for each pixel group in the row direction, for example. For example, for each line in the row direction, a voltage corresponding to the electrical charge accumulated in each pixel is supplied to the vertical signal line. The vertical signal lines are selectively electrically connected to the horizontal signal line. By performing readout operation for each line in the row direction described above sequentially in the vertical direction, the light receiving results of the plurality of pixels arranged two-dimensionally can be read out.

By capturing (reading out) the light receiving results of the returning light using the rolling shutter method for this type of image sensor 51, the light receiving image corresponding to a desired virtual opening (aperture) shape extending in the row direction is acquired. Such control is disclosed in, for example, U.S. Patent No. 8237835.

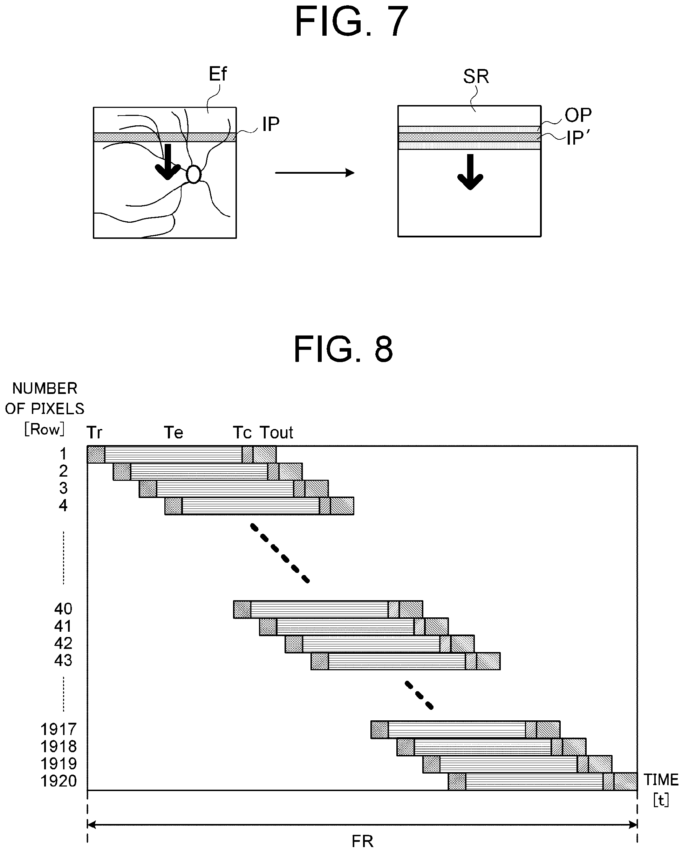

FIG. 7 shows a diagram explaining an operation of the ophthalmic apparatus 1 according to the embodiments. FIG. 7 schematically represents an irradiated range IP of the slit-shaped illumination light irradiated on the fundus Ef and a virtual opening range OP on the light receiving surface SR of the image sensor 51.

For example, the controller 100 described below deflects the slit-shaped illumination light formed by the illumination optical system 20, using the optical scanner 30. Thereby, the irradiated range IP of the slit-shaped illumination light is sequentially moved in a slit direction (for example, the vertical direction) orthogonal to the slit direction (for example, the row direction, the horizontal direction) on the fundus Ef.

On the light receiving surface SR of the image sensor 51, by changing the pixels to be read out in units of lines by the controller 100 described below, the virtual opening range OP is set. The opening range OP is preferable to be the light receiving range IP' of the returning light of the illumination light on the light receiving surface SR or wider than the light receiving range IP'. The controller 100 described below performs the movement control of the opening range OP in synchronization with the movement control of the irradiated range IP of the illumination light. Thereby, without being affected by unnecessary scattered light, high quality images of the fundus Ef with strong contrast can be acquired using a simple configuration.

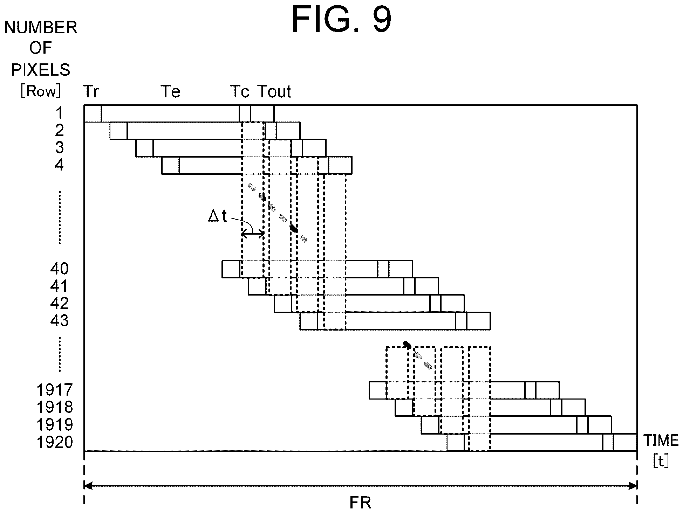

FIG. 8 and FIG. 9 schematically show examples of a control timing of the rolling shutter method for the image sensor 51. FIG. 8 represents an example of a timing of the readout control for the image sensor 51. FIG. 9 represents a timing of the movement control for the irradiated range IP (the light receiving range IP') superimposed on the timing of the readout control in FIG. 8. In FIG. 8 and FIG. 9, the horizontal axis represents the number of rows in the image sensor 51, and the vertical axis represents time.

In addition, in FIG. 8 and FIG. 9, for convenience of explanation, it is assumed that the number of rows in the image sensor 51 is 1920. However, the configuration according to the embodiments is not limited to the number of rows. Further, in FIG. 9, for convenience of explanation, it is assumed that the slit width (width in the row direction) of the slit-shaped illumination light is 40 rows.

The readout control in the row direction includes a reset control, an exposure control, a charge transfer control, and an output control. The reset control is a control that initializes an amount of electrical charge accumulated in the pixels in the row direction. The exposure control is a control that illuminates light on the photodiode and accumulates the electrical charge corresponding to the amount of received light in the capacitor. The charge transfer control is a control that transfers the amount of the electrical charge accumulated in the pixel to the vertical signal line. The output control is a control that outputs the amount of the electrical charge accumulated in the plurality of vertical signal lines via the horizontal signal line. That is, as shown in FIG. 8, the readout time T for reading out the electrical charge accumulated in the pixels in the row direction is the sum of the time Tr required for the reset control, the time Te (exposure time) required for the exposure control, the time Tc required for the charge transfer control, and the time Tout required for the output control.

In FIG. 8, by shifting the readout start timing (start timing of time Tc) in units of rows, the light receiving results (amount of electrical charge) accumulated in the pixels in a desired range in the image sensor 51 are acquired. For example, in case that the pixel range shown in FIG. 8 is for a single frame of the image, the frame rate FR is determined uniquely.

In the present embodiment, the irradiated position of the illumination light on the fundus Ef, the illumination light having a slit width for a plurality of rows, is sequentially shifted in a direction corresponding to the column direction on the fundus Ef.

For example, as shown in FIG. 9, at each predetermined shift time $\Delta t$, the irradiated position of the illumination light on the fundus Ef is shifted in row units in the direction corresponding to the column direction. The shift time $\Delta t$ is obtained by dividing the exposure time Te of the pixel in the image sensor 51 by the slit width (e.g., 40) of the illumination light ($\Delta t = Te / 40$). Synchronized with this movement timing of this irradiated position, the readout start timing of each row of pixels is delayed and is started for each row in units of shift time $\Delta t$. This allows to acquired high quality images of the fundus Ef with strong contrast in a short time with a simple control.

In some embodiments, the image sensor 51 is configured using one or more line sensors.

The eccentric lenses 12A and 12B are examples of the "condensing member" according to the embodiments.

[Configuration of control system]

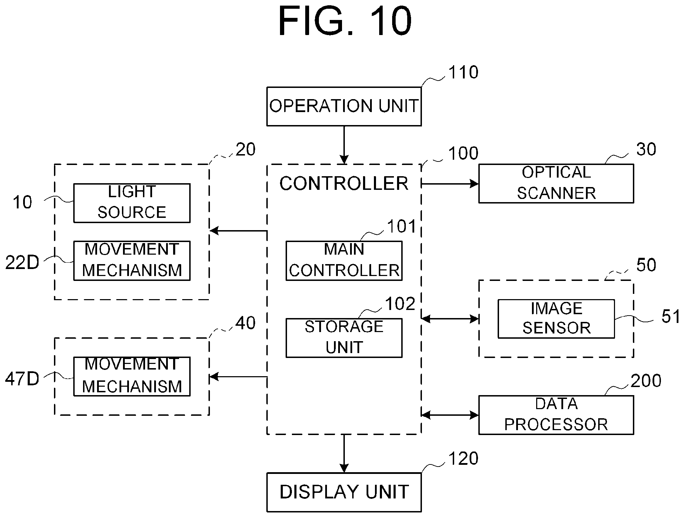

As shown in FIG. 10, the control system of the ophthalmic apparatus 1 is configured with the controller 100 as a center. It should be noted that at least a part of the configuration of the control system may be included in the ophthalmic apparatus 1.

(Controller 100)

The controller 100 controls each part of the ophthalmic apparatus 1. The controller 100 includes a main controller 101 and a storage unit 102. The main controller 101 includes a processor and executes the control processing of each part of the ophthalmic apparatus 1 by executing processing according to the program(s) stored in the storage unit 102.

(Main controller 101)

The main controller 101 performs control for the illumination optical system 20, control for the optical scanner 30, control for the imaging optical system 40, control for the imaging device 50, and control for a data processor 200.
Examples of the control for the illumination optical system 20 include control for the light source 10 and control for a movement mechanism 22D. Examples of the control for the light source 10 include switching the light source on and off (or switching the wavelength region of the light), and change control of the light quantity of the light source. The movement mechanism 22D moves the slit 22 in the optical axis direction of the illumination optical system 20. The main controller 101 controls the movement mechanism 22D in accordance with the state of the eye E to be examined to arrange the slit 22 at the position corresponding to the state of the eye E to be examined. Examples of the state of the eye E to be examined include a shape of the fundus Ef, a dioptric power, and an axial length. The dioptric power can be obtained from a known eye refractive power measurement apparatus as disclosed in Japanese Unexamined Patent Application No. 61- 293430 or Japanese Unexamined Patent Application Publication No. 2010-259495, for example. The axial length can be obtained from a known axial length measurement apparatus or a measurement value acquired by an optical coherence tomography.
For example, the storage unit 102 stores first control information. In the first control information, the positions of the slit 22 on the optical axis of the illumination optical system 20 are associated with the dioptric powers in advance. The main controller 101 identifies the position of the slit 22 corresponding to the dioptric power by referring to the first control information, and controls the movement mechanism 22D so as to arrange the slit 22 at the identified position.
In some embodiments, as the slit 22 moves, the main controller 101 changes the position of the light source 10 and the orientation of the light source 10 in response to changes in the light quantity distribution of the light passing through the aperture formed in the slit 22. In some embodiments, as the slit 22 moves, the main controller 101 changes the position of at least one of the eccentric lens 12A or the eccentric lens 12B and the orientation of at least one of the eccentric lens 12A or the eccentric lens 12B in response to changes in the light quantity distribution of the light passing through the aperture formed in the slit 22.
Examples of the control for the optical scanner 30 include control of a scan range (scan start position and scan end position) and a scan speed.
The control for the imaging optical system 40 includes control for a movement mechanism 47D. The movement mechanism 47D moves the focusing lens 47 in the optical axis direction of the imaging optical system 40. The main controller 101 can control the movement mechanism 47D based on an analysis result of the image acquired using the image sensor 51. Further, the main controller 101 can control the movement mechanism 47D based on a content of operation of the user using an operation unit 110 described below.
Examples of the control for the imaging device 50 include control for the image sensor 51 (rolling shutter control). Examples of the control for the image sensor 51 include the reset control, the exposure control, the charge transfer control, and the output control. Further, the time Tr required for the reset control, the time (exposure time) Te required for the exposure control, the time Tc required for the charge transfer control, and the time Tout required for the output control, etc., can be changed.
Examples of the control for the data processor 200 include various kinds of image processing and various kinds of analysis processing on the light receiving results acquired from the image sensor 51. Examples of the image processing include noise removal processing on the light receiving results, brightness correction processing for easily identifying a predetermined site depicted in the light receiving image based on the light receiving results. Examples of the analysis processing include an identifying processing of the in-focus state.
The data processor 200 can form the light receiving image corresponding to the arbitrary opening range based on the light receiving result(s) read out from the image sensor 51 using the rolling shutter method, under the control from the main controller 101 (controller 100). The data processor 200 can sequentially form light receiving light images corresponding to the opening ranges and can form an image of the eye E to be examined from a plurality of formed light receiving images.
The data processor 200 includes a processor, and realizes the above functions by performing processing corresponding to the program(s) stored in the storage unit or the like.

(Storage unit 102)

The storage unit 102 stores various computer programs and data. The computer programs include an arithmetic program and a control program for controlling the ophthalmic apparatus 1.

(Operation unit 110)

The operation unit 110 includes an operation device or an input device. The operation unit 110 includes buttons and switches (e.g., operation handle, operation knob, etc.) and operation devices (e.g., mouse, keyboard, etc.) provided in the ophthalmic apparatus 1. In addition, the operation unit 110 may include any operation device or any input device, such as a trackball, a control panel, a switch, a button, a dial, etc.

(Display unit 120)

The display unit 120 displays the image of the eye E to be examined generated by data processor 200. The display unit 120 is configured to include a display device such as a flat panel display such as an LCD (Liquid Crystal Display). In addition, the display unit 120 may include various types of display devices such as a touch panel and the like provided in the body of the ophthalmic apparatus 1.

It should be noted that the operation unit 110 and the display unit 120 do not need to be configured to be separate devices. For example, a device like a touch panel, which has a display function integrated with an operation function, can be used. In this case, the operation unit 110 includes the touch panel and a computer program. The operation content for the operation unit 110 is fed to the controller 100 as electrical signal(s). Moreover, operations and inputs of information may be performed using a graphical user interface (GUI) displayed on the display unit 120 and the operation unit 110. In some embodiments, the functions of the display unit 120 and the operation unit 110 are realized a touch screen.

(Other configurations)

In some embodiments, the ophthalmic apparatus 1 further includes a fixation projection system. For example, an optical path of the fixation projection system is coupled with the optical path of the imaging optical system 40 in the configuration of the optical system shown in FIG. 1. The fixation projection system can present internal fixation targets or external fixation targets to the eye E to be examined. In case of presenting the internal fixation target to the eye E to be examined, the fixation projection system includes an LCD that displays the internal fixation target under the control from the controller 100, and projects a fixation light flux output from the LCD onto the fundus Ef of the eye E to be examined. The LCD is configured to be capable of changing the display position of the fixation target on the screen of the LCD. By changing the display position of the fixation target on the screen of the LCD, the projected position of the fixation target on the fundus of the eye E to be examined can be changed. The display position of the fixation target on the LCD can be designated using the operation unit 110 by the user.

In some embodiments, the ophthalmic apparatus 1 includes an alignment system. In some embodiments, the alignment system includes an XY alignment system and a Z alignment system. The XY alignment system is used for position matching between the optical system of the apparatus and the eye E to be examined in a direction intersecting the optical axis of the optical system of the apparatus (objective lens 46). The Z alignment system is used for position matching between the optical system of the apparatus and the eye E to be examined in a direction of the optical axis of the ophthalmic apparatus 1 (objective lens 46).

For example, the XY alignment system projects a bright spot (bright spot in the infrared region or near-infrared region) onto the eye E to be examined. The data processor 200 acquires an anterior segment image of the eye E to be examined on which the bright spot is projected, and obtains the displacement between the bright spot image drawn on the acquired anterior segment image and an alignment reference position. The controller 100 relatively moves the optical system of the apparatus and the eye E to be examined in the direction intersecting the direction of the optical axis so as to cancel the obtained displacement, using the movement mechanism.

For example, the Z alignment system projects alignment light in infrared region or the near-infrared region from a position away from the optical axis of the optical system of the apparatus, and receives the alignment light reflected on the anterior segment of the eye E to be examined. The data processor 200 identifies a distance to the eye E to be examined with respect to the optical system of the apparatus, from the light receiving position of the alignment light that changes in accordance with the distance to the eye E to be examined with respect to the optical system of the apparatus. The controller 100 relatively moves the optical system of the apparatus and the eye E to be examined in the direction of the optical axis using the movement mechanism (not shown) so that the identified distance becomes a predetermined working distance.

In some embodiments, the function of the alignment system is realized by two or more anterior segment cameras positioned at positions away from the optical axis of the optical system of the apparatus. For example, as disclosed in Japanese Unexamined Patent Application Publication No. 2013-248376, the data processor 200 analyzes an anterior segment image of the eye E to be examined substantially simultaneously acquired using the two or more anterior segment cameras, and identifies a three-dimensional position of the eye E to be examined using known trigonometry. The controller 100 controls the movement mechanism (not shown) to relatively move the optical system of the apparatus and the eye E to be examined three-dimensionally so that the optical axis of the optical system of the apparatus substantially coincides with an axis of the eye E to be examined and the distance of the optical system of the apparatus with respect to the eye E to be examined is a predetermined working distance.

[Operation]

Next, the operation of the ophthalmic apparatus 1 will be described.

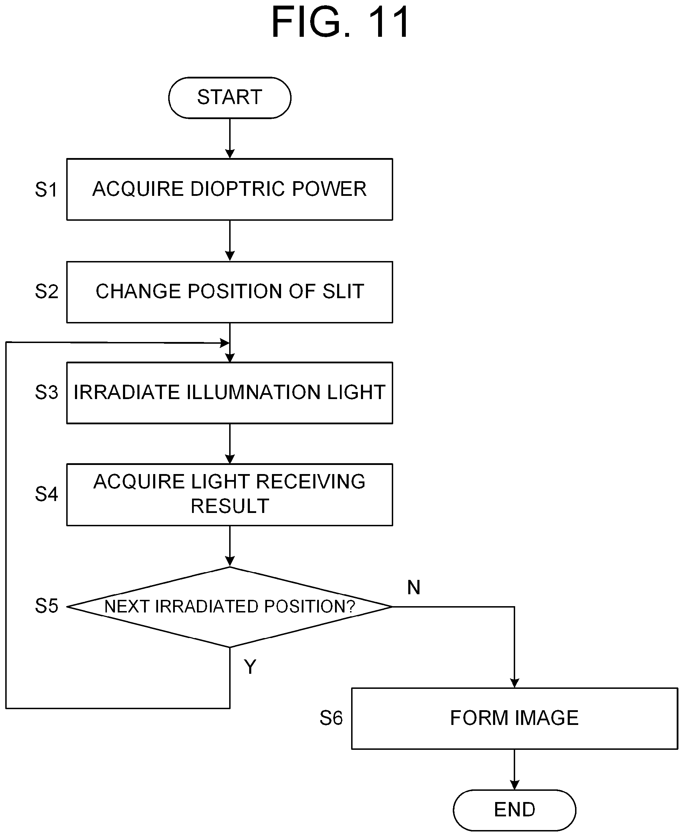

FIG. 11 shows a flowchart of an example of the operation of the ophthalmic apparatus 1 according to the embodiments. The storage unit 102 stores a computer program for realizing the processing shown in FIG. 11. The main controller 101 operates according to the computer program, and thereby the main controller 101 performs the processing shown in FIG. 11.

Here, it is assumed that the alignment of the optical system of the apparatus with respect the eye E to be examined using the alignment system (not shown) has been completed, and that the fixation target is being projected onto the fundus of the eye E to be examined so as to guide the eye E to be examined to a desired fixation position using the fixation projection system (not shown).

(S1: Acquire dioptric power)

First, the main controller 101 acquires the dioptric power of the eye E to be examined from an external ophthalmic measurement apparatus or an electronic medical record.

For example, the main controller 101 acquires the dioptric power of the eye E to be examined from the external ophthalmic measurement apparatus or the electronic medical record via a communication unit (not shown).

(S2: Change position of slit)

Next, the main controller 101 changes the position of the slit 22 on the optical axis of the illumination optical system 20 in accordance with the dioptric power of the eye E to be examined acquired in step S1.

Specifically, the main controller 101 identifies the position of the slit 22 corresponding to the dioptric power by referring to the first control information stored in the storage unit 102, and controls the movement mechanism 22D so as to arrange the slit 22 at the identified position.

(S3: Irradiate illumination light)

Next, the main controller 101 controls the illumination optical system 20 to generate the slit-shaped illumination light, and to start the deflection control of the optical scanner 30 to start irradiating the illumination light onto a desired irradiated region on the fundus Ef. When the irradiation of the illumination light is started, the slit-shaped illumination light is sequentially irradiated within the desired irradiated range as described above.

(S4: Acquire light receiving result)

The main controller 101 acquires the light receiving result(s) of the pixels in the opening range of the image sensor 51 corresponding to the irradiated range of the illumination light on the fundus Ef performed in step S3, as described above.

(S5: Next irradiated position?)

The main controller 101 determines whether or not the next irradiated position is to be irradiated with the illumination light. The main controller 101 can determine whether or not the next irradiated position is to be irradiated with the illumination light, by determining whether or not the irradiated range of the illumination light that is moved sequentially has covered a predetermined imaging range of the fundus Ef.

When it is determined that the next irradiated position is to be irradiated with the illumination light (S5: Y), the operation of the ophthalmic apparatus 1 proceeds to step S3. When it is determined that the next irradiated position is not to be irradiated with the illumination light (S5: N), the operation of the ophthalmic apparatus 1 proceeds to step S6.

(S6: Form image)

In step S5, when it is determined that the next irradiated position is not to be irradiated with the illumination light (S5: N), the main controller 101 controls the data processor 200 to form the image of the eye E to be examined from the light receiving results acquired repeatedly while changing the irradiated range of the illumination light in step S4.

For example, the data processor 200 syntheses a plurality of light receiving results with different irradiated ranges (opening ranges on the light receiving surface SR of the image sensor 51) of the illumination light for the number of times repeating the process in steps S3 to S5, based on the order of the movement of the irradiated range. Thereby, the fundus image of the fundus Ef for one frame is formed.

In some embodiments, in step S3, the illumination light is irradiated on the irradiated range set so as to have an overlapping region with the adjacent irradiated range. Thereby, in step S6, the fundus image for one frame is formed by synthesizing the overlapping regions so as to overlap with each other.

This terminates the operation of the ophthalmic apparatus 1 (END).

<First modification example>

The configuration of the ophthalmic apparatus according to the embodiments is not limited to the above configuration. For example, the ophthalmic apparatus according to a first modification example of the embodiments can illuminate the fundus Ef with two or more illumination light whose central wavelengths are different from each other to acquire images of the fundus Ef.

In the following, the configuration of the ophthalmic apparatus according to the first modification example of the embodiments will be described focusing on the differences from the configuration of the ophthalmic apparatus 1 according to the embodiments.

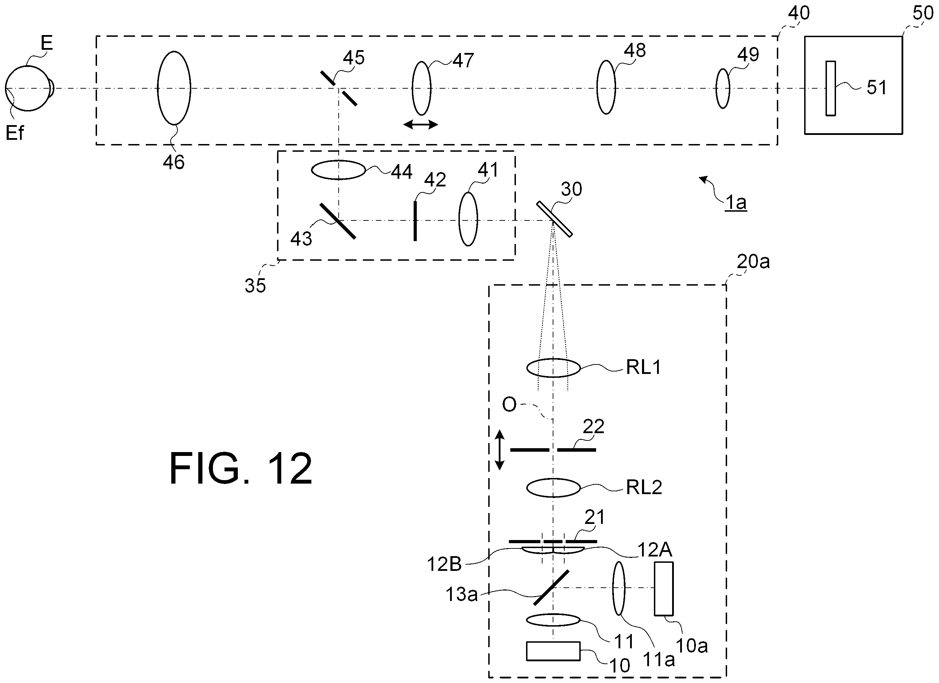

FIG. 12 shows an example of a configuration of the ophthalmic apparatus according to the first modification example of the embodiments. In FIG. 12, parts similar to those in FIG. 1 are denoted by the same reference symbols, and description thereof is omitted as appropriate.

The configuration of an ophthalmic apparatus 1a according to the first modification example of the embodiments is mainly different from that of the ophthalmic apparatus 1 according to the embodiments in that an illumination optical system 20a is provided instead of the illumination optical system 20.

The configuration of the illumination optical system 20a differs from that of the illumination optical system 20 in that a light source 10a, a condenser lens 11a, and a dichroic mirror 13a are added. The dichroic mirror 13a is positioned between the condenser lens 11 and the eccentric lenses 12A and 12B, and couples an optical path of light from the light source 10a with the optical path of the light from the light source 10. The condenser lens 11a is positioned between the light source 10a and the dichroic mirror 13a. The light source 10a is positioned at a front focal position of the condenser lens 11a.

In this case, the images of the fundus Ef are acquired using the illumination light from the light source 10a, using the same rolling shutter method as in the embodiments.

In some embodiments, the front focal distance of the condenser lens 11a is approximately the same as the front focal distance of the condenser lens 11, the emission diameters Sx and Sy of the light source 10 are approximately the same as the emission diameters Sx and Sy of the light source 10a, respectively, and the emitted half-value at half angle θ of the light source 10 is approximately the same as the emitted half-value at half angle θ of the light source 10a. Thereby, the slit 22 can be efficiently illuminated evenly with the illumination light from the light source 10a, as in the embodiments.

In some embodiments, the ophthalmic apparatus 1a is configured to switch between the illumination light from the light source 10 and the illumination light from the light source 10a, and to irradiate onto the iris aperture 21.

<Second modification example>

The configuration of the ophthalmic apparatus according to the embodiments is not limited to the configuration according to the embodiments or the first modification example thereof. For example, in the ophthalmic apparatus according to a second modification example of the embodiments, a prism is used as the condensing member.

In the following, the configuration of the ophthalmic apparatus according to the second modification example of the embodiments will be described focusing on the differences from the configuration of the ophthalmic apparatus 1 according to the embodiments.

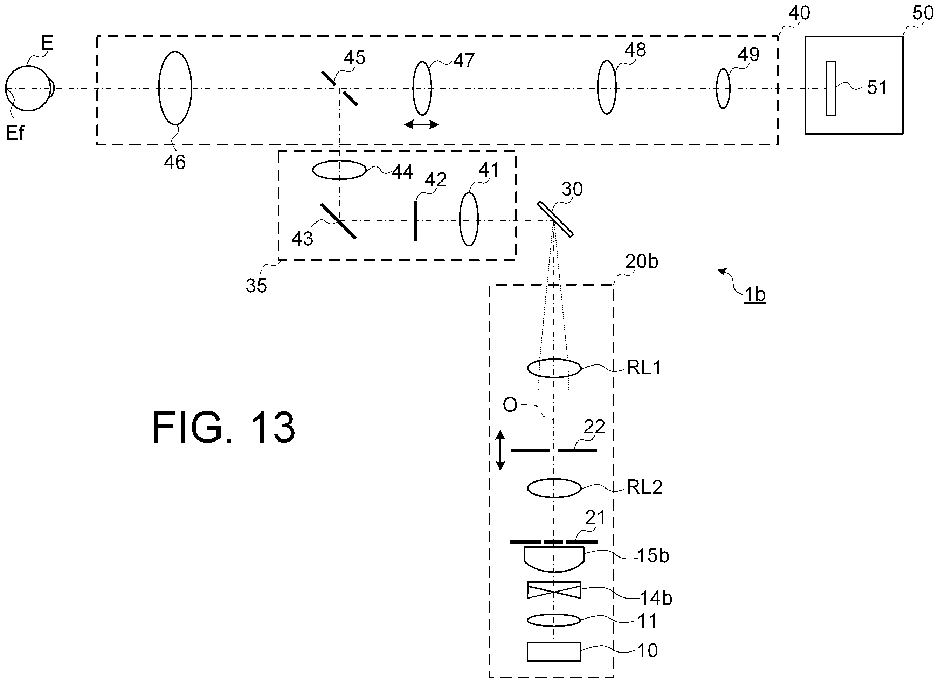

FIG. 13 shows an example of the configuration of the ophthalmic apparatus according to the second modification example of the embodiments. In FIG. 13, like reference numerals designate like parts as in FIG. 1, and the same description may not be repeated.

The configuration of an ophthalmic apparatus 1b according to the second modification example of the embodiments is mainly different from that of the ophthalmic apparatus 1 according to the embodiments in that an illumination optical system 20b is provided instead of the illumination optical system 20.

The configuration of illumination optical system 20b differs from the configuration of the illumination optical system 20 in that a prism 14b and a lens 15b are provided instead of the eccentric lens 12a and 12b. The prism 14b is positioned between the condenser lens 11 and the iris aperture 21. The lens 15b is positioned between the prism 14b and the iris aperture 21.

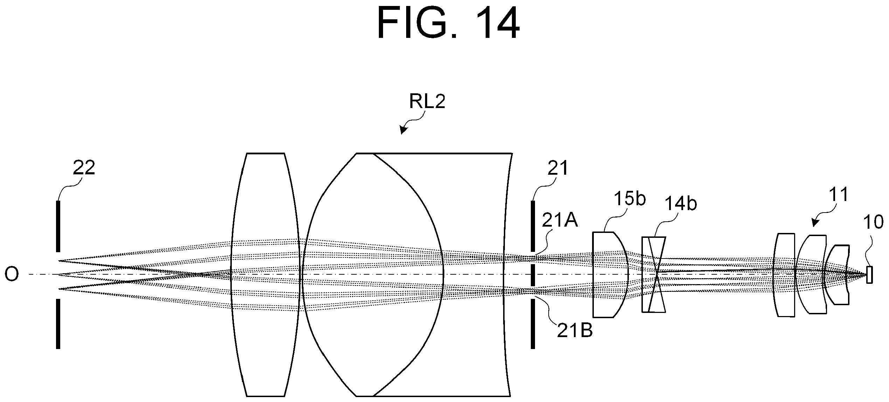

FIG. 14 schematically shows a broad outline of a configuration of the prism 14b and the lens 15b in the Y cross-section. In FIG. 14, like reference numerals designate like parts as in FIG. 4 or FIG. 13. The same description may not be repeated.

The prism 14b has deflected surfaces for the number or the apertures formed in the iris aperture 21, and deflects the illumination light on each deflected surface toward the apertures corresponding to the deflected surfaces. Here, the prism 14b has a first deflected surface and a second deflected surface. The prism 14b deflects the illumination light from the light source 10 (condenser lens 11) on the first deflected surface toward the aperture 21A formed in the iris aperture 21, and deflects the illumination light from the light source 10 (condenser lens 11) on the second deflected surface toward the aperture 21B formed in the iris aperture 21.

The lens 15b condenses the illumination light, that is deflected on the deflected surfaces of the prism 14b, on the apertures corresponding to the deflected surfaces formed in iris aperture 21. Specifically, the lens 15b condenses the illumination light, that is deflected on the first deflected surface of the prism 14b, on the aperture 21A formed in the iris aperture 21, and condenses the illumination light, that is deflected on the second deflected surface of the prism 14b, on the aperture 21B of the iris aperture 21.

In some embodiments, the focal distance of the lens 15b is approximately the same as the focal distance of the eccentric lens 12A (12B). Thereby, the slit 22 can be efficiently illuminated evenly with the illumination light from the light source 10, as in the embodiments.

In the second modification example, with the same configuration as in the first modification example, the ophthalmic apparatus can illuminate the fundus Ef with two or more illumination light whose central wavelengths are different from each other to acquire images of the fundus Ef.

[Actions]

The ophthalmic apparatus according to the embodiments will be described.

An ophthalmic apparatus (1, 1a, 1b) according to some embodiments includes a light source (10, 10a), an iris aperture (21), a condensing member (eccentric lens 12a, 12b, prism14b and lens 15b), a slit (22), and an image sensor (51). The light source is configured to be capable of being positioned at a position substantially conjugate optically to an iris of an eye (E) to be examined. The iris aperture is positioned at a position substantially conjugate optically to the light source. In the iris aperture, an aperture (21A, 21B) is formed at an eccentric position from an optical axis (O). Illumination light from the light source passes through the iris aperture. The condensing member is positioned between the light source and the iris aperture. A slit is configured to be capable of being positioned at a position substantially conjugate optically to a fundus (Ef) of the eye to be examined. In the slit, an aperture is formed. The illumination light that has passed through the aperture passes through the slit-shaped aperture. The image sensor is configured to receive returning light from the fundus illuminated by the illumination light having passed through the slit.

According to such a configuration, the aperture formed in the iris aperture is illuminated with high efficiency. Thereby, the slit can be illuminated with highly efficient illumination light having little unevenness, and high quality images of the eye to be examined can be acquired.

In some embodiments, the aperture of the iris aperture is positioned at a focal position of the condensing member.

According to such a configuration, the illumination light is condensed on the aperture formed in the iris aperture. Thereby, the slit can be illuminated with even illumination light, and high quality images of the eye to be examined can be acquired.

In some embodiments, two or more apertures are formed in the iris aperture.

According to such a configuration, the light quantity of the illumination light entering into the eye can be increased. Thereby, higher quality images of the eye to be examined can be acquired.

In some embodiments, the ophthalmic apparatus includes two or more condensing member arranged corresponding to each of the two or more apertures.

According to such a configuration, the aperture formed in the iris aperture can be illuminated with high efficiency while increasing the light quantity of the illumination light entering into the eye. Thereby, the slit can be illuminated with highly efficient illumination light having little unevenness, and higher quality images of the eye to be examined can be acquired.

In some embodiments, the ophthalmic apparatus further includes a lens (eccentric lens 12A, 12B) positioned at an eccentric position from an optical axis of the iris aperture.

According to such a configuration, the aperture formed in the iris aperture can be efficiently illuminated with a simple configuration.

In some embodiments, the lens is positioned so that an optical axis of the lens passes through the aperture of the iris aperture.

According to such a configuration, the aperture formed in the iris aperture can be most efficiently illuminated with a simple configuration.

In some embodiments, two or more apertures are formed in the iris aperture. The ophthalmic apparatus further includes two or more lenses as the condensing member arranged corresponding to each of the two or more apertures. D-cut processing is applied to a peripheral part of each of two lenses adjacent to each other among the two or more lenses.

According to such a configuration, even when component interference occurs between the lenses positioned corresponding to the aperture formed in the iris aperture, the aperture formed in the iris aperture can be efficiently illuminated with a simple configuration.

In some embodiments, the ophthalmic apparatus further includes a condenser lens (11, 11a) configured to make the illumination light from the light source into approximately collimated light, and a relay lens (relay lens system RL2) positioned between the iris aperture and the slit. For a first direction (X direction) corresponding to a longitudinal direction of the slit-shaped aperture, when a length of the slit is Lx, a focal distance of the relay lens is f1, a focal distance of the lens is f2, a focal distance of the condenser lens is f3, and an emitted half-value at half angle of the light source is θ, the above formula (1) is satisfied. Further, for a second direction (Y direction) corresponding to a shorter direction of the slit-shaped aperture, when a length of the slit is Ly and an amount of eccentricity of the aperture relative to an optical axis is h, following formulas (2) to (4) are satisfied.

According to such a configuration, by condensing the illumination light on the aperture formed in the iris aperture while optimizing the size and the cost of the optical system, the slit can be illuminated with even illumination light.

In some embodiments, the condensing member includes a prism (14b) having deflected surfaces for the number of apertures formed in the iris aperture, and each of the deflected surfaces deflecting the illumination light toward corresponding aperture, and a lens (15b) positioned between the prism and the iris aperture.

According to such a configuration, the aperture formed in the iris aperture can be efficiently illuminated with a simple configuration.

In some embodiments, the ophthalmic apparatus further includes an optical scanner (30) configured to deflect the illumination light having passed through the slit and to guide the deflected illumination light to the fundus. The ophthalmic apparatus is configured to capture light receiving result of the returning light acquired by the image sensor in synchronization with a deflection control for the optical scanner.

According to such a configuration, by efficiently entering the light from the light source into the eye to be examined to scan a wide range of the fundus, even when an inexpensive light source with a wide spread angle is used, the illumination intensity required for imaging the fundus can be secured and high quality images of the eye to be examined can be acquired with a simple configuration.

In some embodiments, the ophthalmic apparatus further includes a hole mirror (45) in which a hole is formed at a position substantially conjugate optically to the iris of the eye to be examined, and configured to couple an optical path of the illumination light having passed through the slit with an optical path of returning light from the fundus.

According to such a configuration, the light from the light source can be efficiently entered into the eye to be examined by the pupil division. Therefore, even when an inexpensive light source with a wide spread angle is used, the illumination intensity required for imaging the fundus can be secured and high quality images of the eye to be examined can be acquired without being affected by the state of the eye to be examined, with a simple configuration.

In some embodiments, the ophthalmic apparatus further includes a movement mechanism (22D) configured to move the slit in an optical axis direction according to a state of the eye to be examined.

According to such a configuration, the position of the slit, that is positioned at a position substantially conjugate optically to the fundus of the eye to be examined, is moved according to the state of the eye to be examined. Thereby, the light from the light source light can be efficiently guided to the fundus of the eye to be examined. This allows to efficiently enter the light from the light source into the eye by the pupil division. Therefore, even when an inexpensive light source with a wide spread angle is used, the illumination intensity required for imaging the fundus can be secured and high quality images of the eye to be examined can be acquired without being affected by the state of the eye to be examined, with a simple configuration.

The embodiments or the modification examples thereof described above are merely examples for carrying out the present invention. Those who intend to implement the present invention can apply any modification, omission, addition, or the like within the scope of the gist of the present invention.

In the above embodiments, the ophthalmic apparatus may have arbitrary functions adaptable in the field of ophthalmology. Examples of such functions include an axial length measurement function, a tonometry function, an optical coherence tomography (OCT) function, an ultrasonic inspection, and the like. It should be noted that the axial length measurement function is realized by the OCT, etc. Further, the axial length measurement function may be used to measure the axial length of the eye to be examined by projecting light onto the eye to be examined and detecting the returning light from the fundus while adjusting the position of the optical system in the Z direction (front-back direction) relative to the eye to be examined. The intraocular pressure measurement function is realized by the tonometer, etc.

The OCT function is realized by the OCT apparatus, etc. The ultrasonic inspection function is realized by the ultrasonic diagnosis apparatus, etc. Further, the present invention can also be applied to an apparatus (multifunctional apparatus) having two or more of such functions.

In some embodiments, a program for causing a computer to execute the method of controlling the ophthalmic apparatus described above is provided. Such a program can be stored in any non-transitory computer-readable recording medium. Examples of the recording medium include a semiconductor memory, an optical disk, a magneto-optical disk (CD-ROM, DVD-RAM, DVD-ROM, MO, etc.), a magnetic storage medium (hard disk, floppy (registered trade mark) disk, ZIP, etc.), and the like. The computer program may be transmitted and received through a network such as the Internet, LAN, etc.

[EXPLANATION OF SYMBOLS]

| 1, 1a, 1b | Ophthalmic apparatus |
|---|---|
| 10, 10a | Light source |
| 11, 11a | Condenser lens |
| 12A, 12B | Eccentric lens |
| 13a | Dichroic mirror |
| 14b | Prism |
| 15b | Lens |
| 20, 20a, 20b | Illumination optical system |
| 21 | Iris aperture |
| 21A, 21B | Aperture |
| 22 | Slit |
| 30 | Optical scanner |
| 35 | Projection optical system |
| 40 | Imaging optical system |
| 42 | Black point plate |
| 43 | Reflective mirror |
| 45 | Hole mirror |
| 46 | Objective lens |
| 47 | Focusing lens |
| 49 | Imaging lens |
| 50 | Imaging device |
| 51 | Image sensor |
| E | Eye to be examined |
| Ef | Fundus |
| RL1, RL2 | Relay lens system |

**Claims**

1. An ophthalmic apparatus, comprising:

   a light source configured to be capable of being positioned at a position substantially conjugate optically to an iris of an eye to be examined;
   an iris aperture in which an aperture, through which illumination light from the light source passes, is formed at an eccentric position from an optical axis, and that is positioned at a position substantially conjugate optically to the light source;
   a condensing member positioned between the light source and the iris aperture;
   a slit in which a slit-shaped aperture, through which the illumination light having passed through the aperture passes, is formed, and that is configured to be capable of being positioned at a position substantially conjugate optically to a fundus of the eye to be examined; and
   an image sensor configured to receive returning light from the fundus illuminated by the illumination light having passed through the slit.

2. The ophthalmic apparatus of claim 1, wherein

the aperture of the the iris aperture is positioned at a focal position of the condensing member.

3. The ophthalmic apparatus of claim 1 or 2, wherein
two or more apertures are formed in the iris aperture.

4. The ophthalmic apparatus of claim 3, comprising
two or more condensing member arranged corresponding to each of the two or more apertures.

5. The ophthalmic apparatus of any one of claims 1 to 4, further comprising
a lens positioned at an eccentric position from an optical axis of the iris aperture.

6. The ophthalmic apparatus of claim 5, wherein
the lens is positioned so that an optical axis of the lens passes through the aperture of the iris aperture.

7. The ophthalmic apparatus of claim 1 or 2, wherein

two or more apertures are formed in the iris aperture,
the ophthalmic apparatus further comprising two or more lenses as the condensing member arranged corresponding to each of the two or more apertures, and
D-cut processing is applied to a peripheral part of each of two lenses adjacent to each other among the two or more lenses.

8. The ophthalmic apparatus of any one of claims 5 to 7, further comprising

a condenser lens configured to make the illumination light from the light source into approximately collimated light; and
a relay lens positioned between the iris aperture and the slit, wherein

for a first direction corresponding to a longitudinal direction of the slit-shaped aperture, when a length of the slit is Lx, a focal distance of the relay lens is f1, a focal distance of the lens is f2, a focal distance of the condenser lens is f3, and an emitted half-value at half angle of the light source is $\theta$, a following formula (1) is satisfied, and
[Equation 1]

$$f3 \times \tan\theta > \left(\frac{Lx}{2} \times \frac{f2}{f1}\right) \qquad (1)$$

for a second direction corresponding to a shorter direction of the slit-shaped aperture, when a length of the slit is Ly and an amount of eccentricity of the aperture relative to an optical axis is h, following formulas (2) to (4) are satisfied.

[Equation 2]

$$f3 \times \tan\theta > \left(\frac{Ly}{2} \times \frac{f2}{f1} + h\right) \qquad (2)$$

$$\frac{Dy}{2} < h \qquad (3)$$

$$Dy = Ly \times \frac{f2}{f1} \qquad (4)$$

9. The ophthalmic apparatus of any one of claims 1 to 4, wherein
the condensing member comprises:

a prism having deflected surfaces for the number of apertures formed in the iris aperture, and each of the deflected surfaces deflecting the illumination light toward corresponding aperture; and
a lens positioned between the prism and the iris aperture.

**10.** The ophthalmic apparatus of any one of claims 1 to 9, further comprising

an optical scanner configured to deflect the illumination light having passed through the slit and to guide the deflected illumination light to the fundus, wherein
the ophthalmic apparatus is configured to capture light receiving result of the returning light acquired by the image sensor in synchronization with a deflection control for the optical scanner.

**11.** The ophthalmic apparatus of any one of claims 1 to 10, further comprising
a hole mirror in which a hole is formed at a position substantially conjugate optically to the iris of the eye to be examined, and configured to couple an optical path of the illumination light having passed through the slit with an optical path of returning light from the fundus.

**12.** The ophthalmic apparatus of any one of claims 1 to 11, further comprising
a movement mechanism configured to move the slit in an optical axis direction according to a state of the eye to be examined.

FIG. 1

EP 4 400 031 A1

# FIG. 2

# FIG. 3

# FIG. 4

X CROSS-SECTION

Y CROSS-SECTION

10

11

12A,12B

12A

12B

21

RL2

22

O

# FIG. 5

# FIG. 6

X CROSS-SECTION

Y CROSS-SECTION

EP 4 400 031 A1

# FIG. 7

# FIG. 8

# FIG. 9

# FIG. 10

OPERATION UNIT  110

CONTROLLER  100

OPTICAL SCANNER  30

LIGHT SOURCE  10

MOVEMENT MECHANISM  22D

20

MAIN CONTROLLER  101

IMAGE SENSOR  51

50

STORAGE UNIT  102

MOVEMENT MECHANISM  47D

40

DATA PROCESSOR  200

DISPLAY UNIT  120

# FIG. 11

```
         ┌─────────────┐
         │    START    │
         └──────┬──────┘
                │
                ▼
     ┌──────────────────────────┐
  S1 │   ACQUIRE DIOPTRIC POWER  │
     └──────────┬───────────────┘
                │
                ▼
     ┌──────────────────────────┐
  S2 │  CHANGE POSITION OF SLIT  │
     └──────────┬───────────────┘
                │
                ▼
     ┌──────────────────────────┐
  S3 │ IRRADIATE ILLUMNATION LIGHT │
     └──────────┬───────────────┘
                │
                ▼
     ┌──────────────────────────┐
  S4 │  ACQUIRE LIGHT RECEIVING  │
     │          RESULT          │
     └──────────┬───────────────┘
                │
                ▼
           ◇─────────────◇              N
  S5      ◇ NEXT IRRADIATED POSITION? ◇ ────────┐
           ◇─────────────◇                       │
                │ Y                               ▼
                │                    ┌──────────────────────┐
                │                 S6 │      FORM IMAGE       │
                └────────────────    └──────────┬───────────┘
                                                │
                                                ▼
                                           ┌─────────┐
                                           │   END   │
                                           └─────────┘
```

FIG. 12

FIG. 13

EP 4 400 031 A1

# FIG. 14

EP 4 400 031 A1

## INTERNATIONAL SEARCH REPORT

| International application No. |
|---|
| **PCT/JP2022/025094** |

**A. CLASSIFICATION OF SUBJECT MATTER**

*A61B 3/135*(2006.01)i
FI:  A61B3/135

According to International Patent Classification (IPC) or to both national classification and IPC

**B. FIELDS SEARCHED**

Minimum documentation searched (classification system followed by classification symbols)

A61B3/135

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2000-135200 A (CANON INC.) 16 May 2000 (2000-05-16)<br>fig. 1, 5 | 1-12 |
| A | WO 2007/055668 A2 (OPTOTEK D.O.O.) 18 May 2007 (2007-05-18)<br>fig. 2a-2b | 1-12 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **02 August 2022** | **09 August 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
**Information on patent family members**

International application No.

**PCT/JP2022/025094**

| Patent document cited in search report | | | Publication date (day/month/year) | Patent family member(s) | | | Publication date (day/month/year) |
|---|---|---|---|---|---|---|---|
| JP | 2000-135200 | A | 16 May 2000 | (Family: none) | | | |
| WO | 2007/055668 | A2 | 18 May 2007 | SI | 000022179 | A | |

Form PCT/ISA/210 (patent family annex) (January 2015)

REFERENCES CITED IN THE DESCRIPTION

**Patent documents cited in the description**

- US 7831106 B
- US 8237835 B
- JP 61293430 A
- JP 2010259495 A
- JP 2013248376 A